# EUROPEAN PATENT APPLICATION

(11) **EP 4 098 311 A1**
(43) Date of publication of application: **07.12.2022**
(21) Application number: 20917258.4
(22) Date of filing: 21.12.2020
(51) Int. Cl.: A61M 25/092

(54) **CATHETER**

(30) Priority: 28.01.2020 JP 2020011322
(71) Applicant: Asahi Intecc Co., Ltd., Seto-shi, Aichi 489-0071 (JP)
(72) Inventor: NAMIMA, Satoshi, Seto-shi, Aichi 489-0071 (JP); KUBO, Yuta, Seto-shi, Aichi 489-0071 (JP)
(74) Representative: Winter, Brandl - Partnerschaft mbB
(86) International application number: PCT/JP2020/047630
(87) International publication number: WO 2021/153092

(57) **Abstract**

A catheter includes a catheter shaft, an operating portion coupled to a proximal end portion of the catheter shaft, and at least one operating wire. A distal end portion of each operating wire is fixed to a distal end portion of the catheter shaft, and a proximal end portion of each operating wire is fixed to the operating portion or the proximal end portion of the catheter shaft. The operating portion includes a first rotary member. The first rotary member makes relative rotation with respect to the catheter shaft around an axis parallel to an extending direction of the proximal end portion of the catheter shaft to change a tensile force of the operating wire and thus change a degree of bending of the distal end portion of the catheter shaft.

## Description

### TECHNICAL FIELD

The technique disclosed in this description relates to a catheter.

### BACKGROUND

Catheters insertable into biological lumens are used for examination and treatment of, for example, the heart and digestive organs. There are conventionally known catheters in which an operating portion provided on a proximal end side of a catheter shaft is operated so as to bend a distal end portion of the catheter shaft (see, for example, Patent Literature 1). In this catheter, a knob protruding laterally from the operating portion is operated so that the distal end portion of the catheter shaft may be bent.

### CITATION LIST

### Patent Literature

Patent Literature 1: JP 2014-213173 A

### SUMMARY

### TECHNICAL PROBLEM

In general, the room for examination and treatment is not larger than necessary and therefore, during the examination and treatment using the catheter, the operating portion of the catheter is often located at a position close to the patient's body (e.g., near the thigh). The above conventional catheter has an issue that, for example, when the operating portion is operated to bend the distal end portion of the catheter shaft or the entire catheter is rotated, a part (e.g., knob) of the operating portion located at a position close to the patient's body comes into contact with the patient's body, which results in an undesirable operability.

This description discloses the technique that may provide a solution to the issue described above.

### SOLUTION TO PROBLEM

The technique disclosed in this description may be implemented as the aspects below, for example.
(1) A catheter disclosed in this description is a catheter including a catheter shaft; an operating portion coupled to a proximal end portion of the catheter shaft; and at least one operating wire, a distal end portion of the operating wire being fixed to a distal end portion of the catheter shaft, and a proximal end portion of the operating wire being fixed to the operating portion or the proximal end portion of the catheter shaft, wherein the operating portion includes a first rotary member that makes relative rotation with respect to the catheter shaft around an axis parallel to an extending direction of the proximal end portion of the catheter shaft to change a tensile force of the operating wire and thus change a degree of bending of the distal end portion of the catheter shaft. In this catheter, the first rotary member makes relative rotation with respect to the catheter shaft so as to change the degree of bending of the distal end portion of the catheter shaft in a flexible manner. In this catheter, the operating portion does not include a portion such as a knob protruding laterally from the operating portion, for example, and any portion does not protrude laterally from the operating portion as a result of the operation to change the degree of bending of the distal end portion of the catheter shaft (relative rotation operation of the first rotary member). Therefore, this catheter prevents a part of the operating portion from being in contact with the patient's body even when the operation is performed to change the degree of bending of the distal end portion of the catheter shaft and when the entire catheter is rotated. Therefore, with this catheter, it is possible to control the degree of bending of the distal end portion of the catheter shaft and prevent the accompanying reduction in the operability of the catheter.
(2) In the above catheter, a configuration may be such that the plurality of operating wires is included, wherein the respective distal end portions of the operating wires are fixed to different positions from each other in a circumferential direction in the distal end portion of the catheter shaft, and the operating portion further includes a second rotary member that makes relative rotation with respect to the catheter shaft around the axis to select the operating wire having a largest tensile force among the operating wires and thus change a bending direction of the distal end portion of the catheter shaft. In this catheter, the second rotary member makes relative rotation with respect to the catheter shaft so as to change the bending direction of the distal end portion of the catheter shaft in a flexible manner. In this catheter, any portion does not protrude laterally from the operating portion as a result of the operation to change the bending direction of the distal end portion of the catheter shaft (relative rotation operation of the second rotary member). Therefore, this catheter prevents a part of the operating portion from being in contact with the patient's body even when the operation is performed to change the bending direction of the distal end portion of the catheter shaft. Therefore, with this catheter, it is possible to control the bending direction of the distal end portion of the catheter shaft and prevent the accompanying reduction in the operability of the catheter. Moreover, with this catheter, the degree of bending of the distal end portion of the catheter shaft may be controlled by the relative rotation of the first rotary member, and the bending direction of the distal end portion of the catheter shaft may be controlled by the relative rotation of the second rotary member, and therefore the bending degree and the bending direction of the distal end portion of the catheter shaft may be controlled separately by the two rotary members, which may reduce the allowance for each control and further improve the operability of the catheter as compared to a configuration in which the bending degree and the bending direction are controlled by one common operating member.
(3) In the above catheter, a configuration may be such that the operating wires include at least one pair of the two operating wires having the distal end portions fixed to different positions from each other by 180° in the circumferential direction in the distal end portion of the catheter shaft. With this catheter, the bending direction of the distal end portion of the catheter shaft may be changed to one direction and the direction opposite to the one direction by 180°, which may further improve the operability of the catheter.
(4) In the above catheter, a configuration may be such that the operating portion further includes a swing member that is attached to the proximal end portion of the catheter shaft so as to change an inclination with respect to the axis and that has the proximal end portion of the operating wire fixed thereto, and the first rotary member makes the relative rotation to change a degree of inclination of the swing member and change the tensile force of the operating wire. With this catheter, a relatively simple configuration enables high-accurate control on the degree of bending of the distal end portion of the catheter shaft by the relative rotation of the first rotary member. Moreover, in this catheter, a change in the degree of inclination of the swing member simultaneously causes an increase (tenseness) in the tensile force of one operating wire and a decrease (looseness) in the tensile force of the other operating wire, and therefore the bending of the distal end portion of the catheter shaft may be achieved with a smaller amount of operation (a smaller amount of rotation of the first rotary member), and the operability of the catheter may be further improved.
(5) In the above catheter, a configuration may be such that the second rotary member is located further on a proximal end side than the swing member and is attached so as to make relative movement with respect to the catheter shaft along the axis, a surface of the second rotary member on a side facing the swing member is provided with a protruding portion at a position shifted from the axis, and the first rotary member makes the relative rotation to change a position of the second rotary member along a direction of the axis and thus change the degree of inclination of the swing member. With this catheter, a relatively simple configuration enables high-accurate control on the bending degree and the bending direction of the distal end portion of the catheter shaft by the relative rotation of the first rotary member and the second rotary member.
(6) In the above catheter, a configuration may be such that a shape of the second rotary member when viewed from a distal end side of the catheter shaft is a shape having an outer peripheral line including at least two points having different distances from the axis from each other, and each of the operating wires abuts the outer peripheral line, the operating portion further includes a fixing member that is located further on a proximal end side than the second rotary member, is attached so as to make relative movement with respect to the catheter shaft along the axis, and has the proximal end portion of the operating wire fixed thereto, and the first rotary member makes the relative rotation to change a position of the fixing member along a direction of the axis and thus change the tensile force of the operating wire. With this catheter, a relatively simple configuration enables high-accurate control on the bending degree and the bending direction of the distal end portion of the catheter shaft by the relative rotation of the first rotary member and the second rotary member.
(7) In the above catheter, a configuration may be such that a tube member provided with a plurality of through-holes individually accommodating the respective operating wires is further included, wherein the first rotary member is provided with a first through-hole that penetrates in the direction of the axis and has such a shape that a distance from the axis gradually decreases along the circumferential direction of the catheter shaft when viewed in the direction of the axis, the second rotary member is provided with a second through-hole that penetrates in the direction of the axis and has a shape extending in a radial direction of the catheter shaft when viewed in the direction of the axis, and the tube member is inserted into the first through-hole and the second through-hole. With this catheter, a relatively simple configuration enables high-accurate control on the bending degree and the bending direction of the distal end portion of the catheter shaft by the relative rotation of the first rotary member and the second rotary member.

The technique disclosed in this description may be achieved in various aspects and, for example, may be achieved in the form of a catheter, a manufacturing method thereof, etc.

### BRIEF DESCRIPTION OF DRAWINGS

Fig. 1 is an explanatory diagram schematically illustrating a configuration of a catheter 100 according to a first embodiment.
Fig. 2 is an explanatory diagram schematically illustrating the configuration of the catheter 100 according to the first embodiment.
Fig. 3 is an explanatory diagram illustrating a cross-sectional configuration of a catheter shaft 110.
Fig. 4 is an explanatory diagram illustrating the cross-sectional configuration of the catheter shaft 110.
Fig. 5 is an explanatory diagram illustrating an operation of the catheter 100 according to the first embodiment.
Fig. 6 is an explanatory diagram illustrating the operation of the catheter 100 according to the first embodiment.
Fig. 7 is an explanatory diagram schematically illustrating a configuration of a catheter 200 according to a second embodiment.
Fig. 8 is an explanatory diagram schematically illustrating the configuration of the catheter 200 according to the second embodiment.
Fig. 9 is an explanatory diagram schematically illustrating the configuration of the catheter 200 according to the second embodiment.
Fig. 10 is an explanatory diagram schematically illustrating a configuration of a catheter 300 according to a third embodiment.
Fig. 11 is an explanatory diagram schematically illustrating the configuration of the catheter 300 according to the third embodiment.
Fig. 12 is an explanatory diagram illustrating a cross-sectional configuration of a tube member 370.
Fig. 13 is an explanatory diagram illustrating an operation of the catheter 300 according to the third embodiment.
Fig. 14 is an explanatory diagram illustrating the operation of the catheter 300 according to the third embodiment.
Fig. 15 is an explanatory diagram illustrating the operation of the catheter 300 according to the third embodiment.

### EMBODIMENTS

### A. First Embodiment:

### A-1. Basic Configuration of Catheter 100:

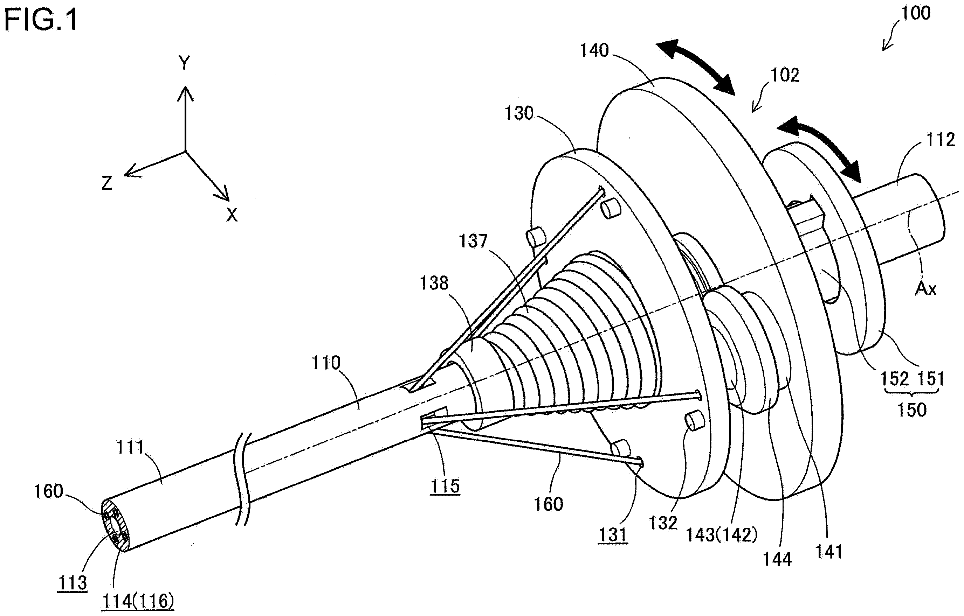

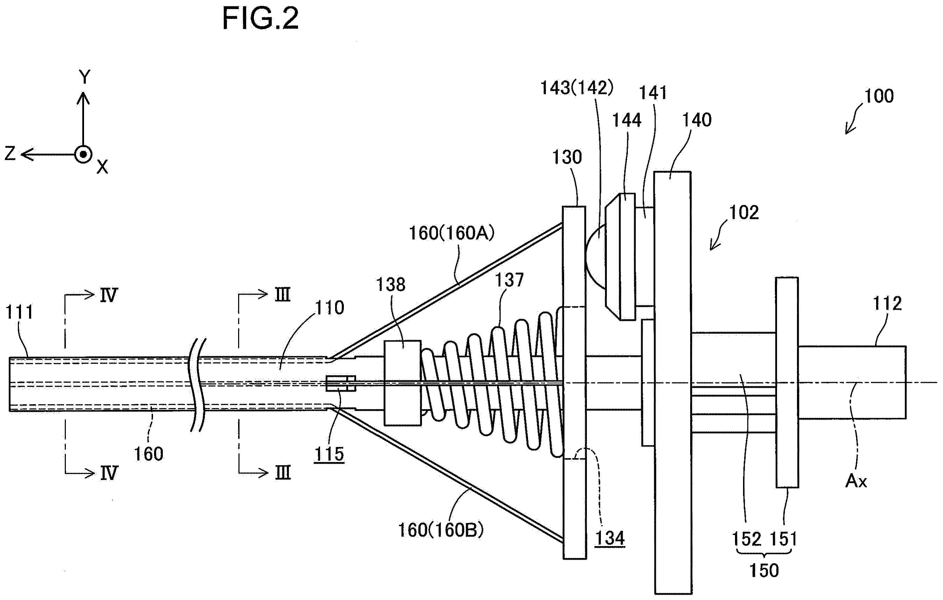

Figs. 1 and 2 are explanatory diagrams schematically illustrating a configuration of the catheter 100 according to a first embodiment. Figs. 1 and 2 illustrate an external configuration of the catheter 100 together with XYZ axes that are perpendicular to each other. In Figs. 1 and 2, a Z-axis positive-direction side is a distal end side (far side) inserted into the body, and a Z-axis negative-direction side is a proximal end side (near side) operated by an operator such as a doctor. This also applies to the other figures. In this description, with regard to the catheter and each component thereof, an end on the distal end side is referred to as "distal end", the distal end and its vicinity as "distal end portion", an end on the proximal end side as "proximal end", and the proximal end and its vicinity as "proximal end portion".

The catheter 100 is a device inserted into a biological lumen and used for examination and treatment of, for example, the heart and digestive organs. Here, the biological lumen includes various lumens of blood vessel-related, lymph gland-related, biliary tract-related, urinary tract-related, airway-related, digestive organ-related, secretory glands, and reproductive organs.

As illustrated in Figs. 1 and 2, the catheter 100 includes a catheter shaft 110, an operating portion 102, and four operating wires 160. In Figs. 1 and 2, a part of the catheter shaft 110 is not illustrated.

The catheter shaft 110 is a tubular member inserted into a biological lumen of a patient. The outer shape of the catheter shaft 110 in cross-section (XY cross-section) may be any shape, e.g., a circular shape. The outer diameter of the catheter shaft 110 is, for example, approximately 0.3 mm to 2.0 mm. Figs. 1 and 2 illustrate a state where the catheter shaft 110 is in a straight line substantially parallel to the Z-axis direction, but the catheter shaft 110 is flexible enough to be curved.

The catheter shaft 110 is made of, for example, a resin material or metallic material. Examples of the resin material for forming the catheter shaft 110 may include polyamide resin, polyolefin resin, polyester resin, polyurethane resin, silicone resin, and fluorine resin. Examples of the metallic material for forming the catheter shaft 110 may include stainless steel such as SUS304, NiTi alloy, and cobalt-chromium alloy. The entire catheter shaft 110 may be made of the identical material, or a part of the catheter shaft 110 may be made of a different material from those of other parts.

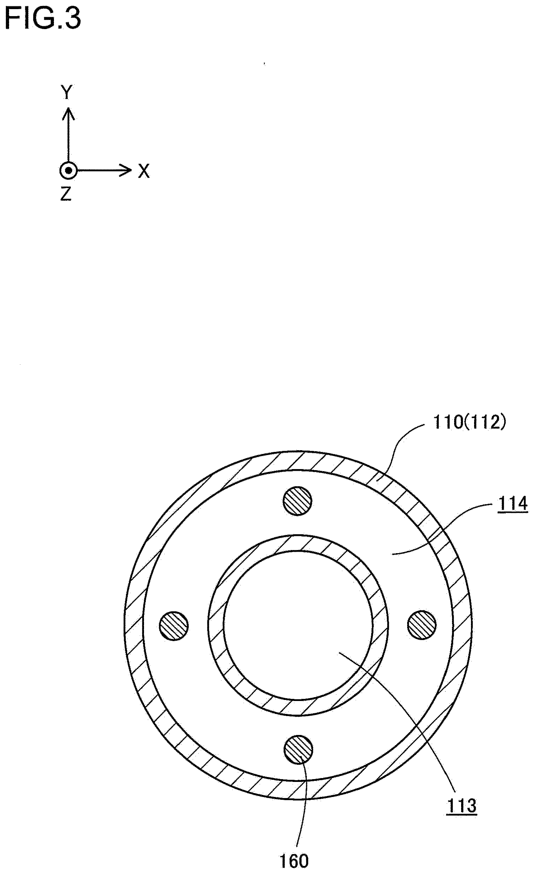

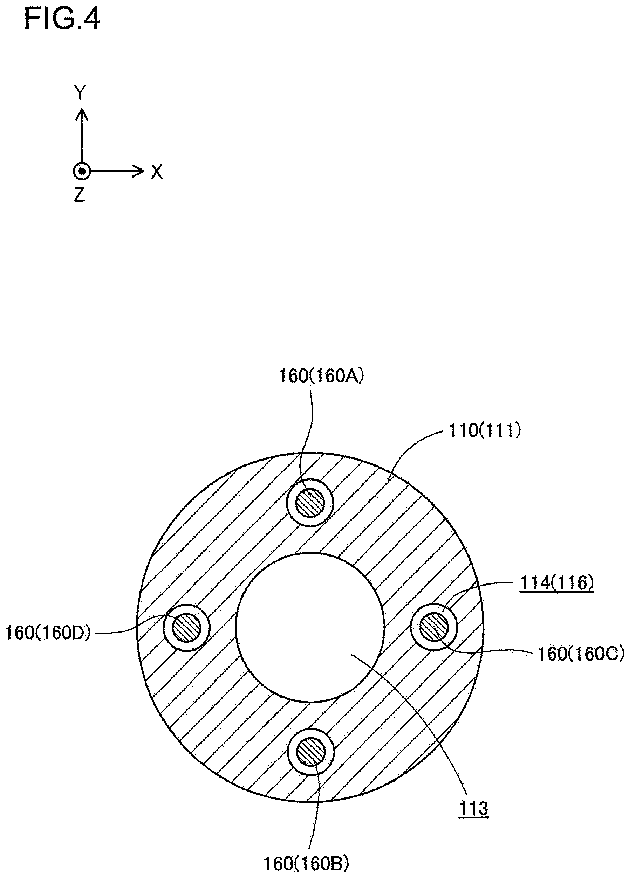

Figs. 3 and 4 are explanatory diagrams illustrating a cross-sectional configuration of the catheter shaft 110. Fig. 3 illustrates a configuration of the catheter shaft 110 in cross-section (XY cross-section) at a position of III-III in Fig. 2 (a position of a proximal end portion 112), and Fig. 4 illustrates a configuration of the catheter shaft 110 in cross-section (XY cross-section) at a position of IV-IV in Fig. 2 (a position of a distal end portion 111). As illustrated in Figs. 3 and 4, the catheter shaft 110 is provided with a through-hole 113 extending in an axial direction from the distal end to the proximal end. The space formed by the through-hole 113 functions as, for example, a lumen for inserting the guide wire or distributing the medical liquid. According to the present embodiment, in cross-section of the catheter shaft 110, the through-hole 113 is a concentric circle with respect to the center of the catheter shaft 110.

The catheter shaft 110 is provided with a through-hole 114 extending in the axial direction from the distal end to the proximal end. The space formed by the through-hole 114 functions as a lumen for accommodating each of the operating wires 160. As illustrated in Fig. 3, in cross-section of the catheter shaft 110, the through-hole 114 is a concentric circle with respect to the center of the catheter shaft 110 and has an annular shape surrounding the through-hole 113. As illustrated in Fig. 4, in the distal end portion 111 of the catheter shaft 110, the through-hole 114 is split into four through-holes 116 that are independent of each other. In cross-section of the distal end portion 111 of the catheter shaft 110, each of the through-holes 116 is circular and is formed at a position shifted by 90° along a circumferential direction of the catheter shaft 110.

As illustrated in Figs. 1 and 2, a side surface of the proximal end portion 112 of the catheter shaft 110 is provided with four wire extraction holes 115 connecting the through-hole 114 and the external space. The four wire extraction holes 115 are formed at positions shifted by 90° along the circumferential direction of the catheter shaft 110 to correspond to the four through-holes 116 formed in the distal end portion 111 of the catheter shaft 110.

Each of the operating wires 160 is a member provided to bend the distal end portion 111 of the catheter shaft 110. Each of the operating wires 160 is made of, for example, a metallic material. Examples of the metallic material for forming each of the operating wires 160 may include stainless steel such as SUS304, NiTi alloy, and cobalt-chromium alloy.

Each of the operating wires 160 is accommodated in the through-hole 114 formed in the catheter shaft 110. As described above, in the distal end portion 111 of the catheter shaft 110, the through-hole 114 is split into the four through-holes 116 that are independent of each other, and therefore each of the four operating wires 160 is accommodated in the corresponding through-hole 116 among the four through-holes 116 (see Fig. 4). The distal end portion of each of the operating wires 160 is fixed to the distal end portion 111 of the catheter shaft 110 in a state where it is accommodated in the corresponding through-hole 116. Specifically, the catheter 100 according to the present embodiment includes two pairs of the two operating wires 160 (a pair of operating wires 160A and 160B and a pair of operating wires 160C and 160D illustrated in Fig. 4) having the distal end portion thereof fixed to different positions from each other by 180° in the circumferential direction in the distal end portion 111 of the catheter shaft 110.

Each of the operating wires 160 is accommodated in the single through-hole 114 in portions other than the distal end portion 111 of the catheter shaft 110 (see Fig. 3). As illustrated in Figs. 1 and 2, in the proximal end portion 112 of the catheter shaft 110, the proximal end portion of each of the operating wires 160 is extracted to the external space through the corresponding wire extraction hole 115 among the four wire extraction holes 115 formed on the side surface of the catheter shaft 110 and is fixed to the operating portion 102 (more specifically, a swing member 130, described below, included in the operating portion 102).

The operating portion 102 is a portion to perform the operation to bend the distal end portion 111 of the catheter shaft 110 and is coupled to the proximal end portion 112 of the catheter shaft 110. The operating portion 102 includes the swing member 130, a first rotary member 150, and a second rotary member 140. Each portion included in the operating portion 102 is made of, for example, a resin material or a metallic material. Examples of the resin material for forming each portion included in the operating portion 102 may include polyamide resin, polyolefin resin, polyester resin, polyurethane resin, silicone resin, and fluorine resin. Examples of the metallic material for forming each portion included in the operating portion 102 may include stainless steel such as SUS304, NiTi alloy, and cobalt-chromium alloy. The entire portions included in the operating portion 102 may be made of the identical material, or a part of the portions included in the operating portion 102 may be made of a material different from that of the other parts.

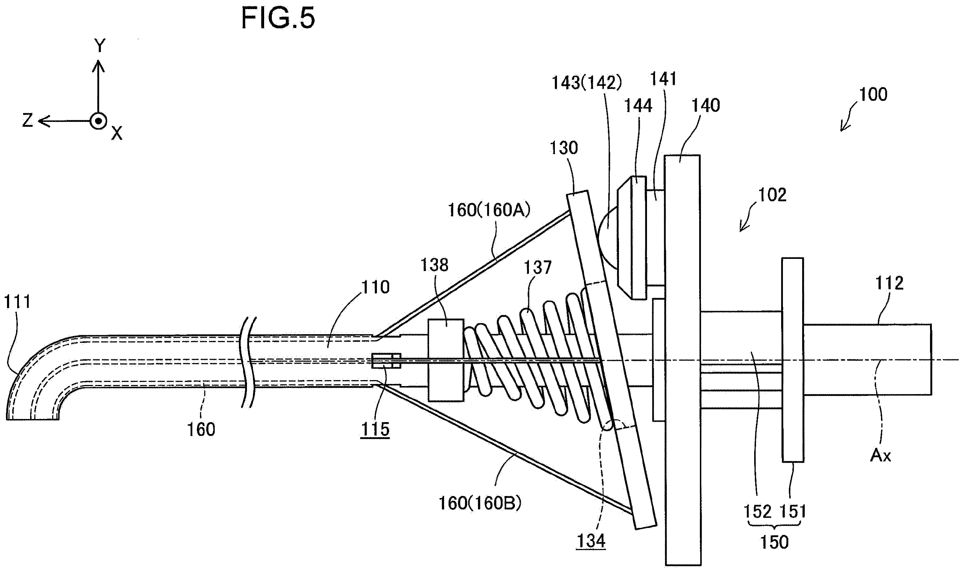

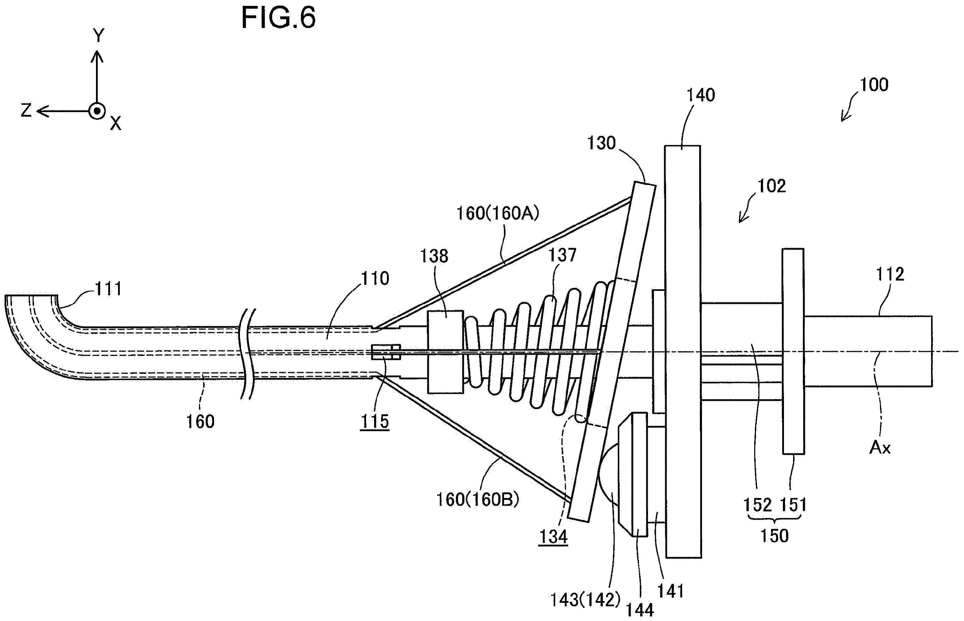

The swing member 130 is a member having substantially a disc-like shape and is attached to the proximal end portion 112 of the catheter shaft 110 so as to change the inclination with respect to an axis (hereinafter simply referred to as "axis Ax") parallel to the extending direction of the proximal end portion 112. More specifically, the swing member 130 is provided with a shaft insertion hole 134 (indicated in a dashed line in Fig. 2) that is a through-hole through which the catheter shaft 110 is inserted. The inner diameter of the shaft insertion hole 134 is larger than the outer diameter of the catheter shaft 110 enough not to prevent changes in the inclination of the swing member 130. The swing member 130 is fixed to a proximal end portion of a spring member 137 that is located further on the distal end side than the swing member 130 and is wound around the catheter shaft 110. A distal end portion of the spring member 137 is fixed to the catheter shaft 110 by a fixing member 138. Therefore, the swing member 130 is swingable due to the elastic force of the spring member 137 with the position of the fixing member 138 as a fulcrum to thus change the inclination of the swing member 130 with respect to the axis Ax (see Figs. 5 and 6 described below). In the basic state of the swing member 130 illustrated in Fig. 2 (the state in which no force from the second rotary member 140 described below acts on the swing member 130), the surfaces of the swing member 130 on the distal end side and the proximal end side are substantially perpendicular to the axis Ax.

The proximal end portion of each of the operating wires 160 is fixed to the swing member 130. More specifically, the swing member 130 is provided with four wire insertion holes 131 that are through-holes through which the operating wires 160 are inserted. The four wire insertion holes 131 are formed at positions shifted by 90° along the circumferential direction of the catheter shaft 110 to correspond to the four wire extraction holes 115 formed on the side surface of the catheter shaft 110. The proximal end portion of each of the operating wires 160 is inserted into the corresponding wire insertion hole 131 among the four wire insertion holes 131 formed in the swing member 130, then folded back, inserted into another wire insertion hole, not illustrated, formed in the swing member 130, and fixed to the swing member 130 by a fixing tool 132 in the state where the initial tensile force has been adjusted.

The second rotary member 140 is a member having substantially a disc-like shape and is located further on the proximal end side than the swing member 130 in the proximal end portion 112 of the catheter shaft 110. The second rotary member 140 is attached so as to make relative rotation with respect to the catheter shaft 110 and the first rotary member 150 around the axis Ax and make relative movement with respect to the catheter shaft 110 along the axis Ax.

A surface of the second rotary member 140 on the side facing the swing member 130 is provided with a protruding portion 143 at a position shifted from the axis Ax in the radial direction of the catheter shaft 110. More specifically, the surface of the second rotary member 140 on the side facing the swing member 130 is provided with a receiving portion 141 that protrudes to the distal end side and is provided with a recessed portion on the surface facing the swing member 130, and the recessed portion of the receiving portion 141 accommodates a ball 142 having a spherical shape. A cover member 144 is attached to a distal end of the receiving portion 141 in the state where the ball 142 is accommodated in the recessed portion of the receiving portion 141. The cover member 144 is provided with a through-hole, and a portion of the ball 142 protruding outward through the through-hole formed in the cover member 144 forms the protruding portion 143. When viewed in the direction of the axis Ax, the distal end of the protruding portion 143 is formed in such a position that it is overlapped with the swing member 130.

The first rotary member 150 is located further on the proximal end side than the second rotary member 140 in the proximal end portion 112 of the catheter shaft 110. The first rotary member 150 includes a disc portion 151 having substantially a disc-like shape and a hollow cylindrical portion 152 that has substantially a hollow cylindrical shape and is provided on the distal end side of the disc portion 151. The distal end portion of the hollow cylindrical portion 152 is inserted into a through-hole formed in the second rotary member 140, and the first rotary member 150 is coupled to the second rotary member 140 so as to make relative rotation with respect to the second rotary member 140. The first rotary member 150 and the second rotary member 140 may be disconnected. The first rotary member 150 is provided with a through-hole, and an inner peripheral surface of the through-hole is provided with a spirally-arranged protruding portion or recessed portion. An outer peripheral surface of the catheter shaft 110 is provided with a recessed portion or protruding portion that engage with the protruding portion or recessed portion on the inner peripheral surface of the above-described through-hole of the first rotary member 150. As the first rotary member 150 and the catheter shaft 110 have this configuration, the first rotary member 150 makes relative rotation with respect to the catheter shaft 110 and thus makes relative movement with respect to the catheter shaft 110 along the axis Ax. This configuration allows the first rotary member 150 to maintain the state of having made relative movement along the axis Ax. When the first rotary member 150 makes relative movement along the axis Ax while making relative rotation with respect to the catheter shaft 110, the second rotary member 140 accordingly makes relative movement along the axis Ax without making relative rotation with respect to the catheter shaft 110.

### A-2. Operation of Catheter 100:

Figs. 5 and 6 are explanatory diagrams illustrating an operation of the catheter 100 according to the first embodiment. In the catheter 100 according to the present embodiment, the second rotary member 140 and the first rotary member 150 of the operating portion 102 are operated so that the distal end portion 111 of the catheter shaft 110 may be bent in a flexible manner.

Fig. 2 illustrates the basic state of the catheter 100. In the state illustrated in Fig. 2, when viewed in the direction of the axis Ax, the position (hereinafter simply referred to as "circumferential position") of the protruding portion 143 of the second rotary member 140 along the circumferential direction of the catheter shaft 110 substantially matches the circumferential position of one (the operating wire 160A) of the four operating wires 160 at the fixed position on the swing member 130 (both of which are at the position in the Y-axis positive direction when viewed from the axis Ax). As described above, in the basic state illustrated in Fig. 2, no force from the second rotary member 140 acts on the swing member 130, and the surfaces of the swing member 130 on the distal end side and the proximal end side are substantially perpendicular to the axis Ax. In this state, the tensile force of a predetermined value or more does not act on any of the four operating wires 160, and the distal end portion 111 of the catheter shaft 110 is not bent.

For example, as illustrated in Fig. 5, in the basic state, the first rotary member 150 makes relative rotation with respect to the catheter shaft 110 and thus the first rotary member 150 makes relative movement to the distal end side with respect to the catheter shaft 110, and accordingly the second rotary member 140 also makes relative movement to the distal end side with respect to the catheter shaft 110. This movement of the second rotary member 140 causes the protruding portion 143 of the second rotary member 140 to come into contact with the surface of the swing member 130 on the side facing the second rotary member 140 and press the swing member 130 to the distal end side. Here, the protruding portion 143 of the second rotary member 140 is formed at a position shifted from the axis Ax. The swing member 130 is attached to the proximal end portion 112 of the catheter shaft 110 so as to change the inclination with respect to the axis Ax. Therefore, the swing member 130 pressed by the protruding portion 143 of the second rotary member 140 is inclined with respect to the axis Ax such that a portion (hereinafter referred to as "contact portion") in contact with the protruding portion 143 of the second rotary member 140 is displaced to the distal end side and a portion (hereinafter referred to as "opposite portion") shifted by 180° with respect to the contact portion in the circumferential direction of the catheter shaft 110 is displaced to the proximal end side. Accordingly, there is a decrease in the tensile force of the operating wire 160 (the operating wire 160A in the example of Fig. 5 and hereinafter referred to as "loosened operating wire") fixed closest to the contact portion in the swing member 130 among the four operating wires 160, while there is an increase in the tensile force of the operating wire 160 (the operating wire 160B in the example of Fig. 5 and hereinafter referred to as "tense operating wire") fixed closest to an opposite portion in the swing member 130. As a result, the tensile force of the tense operating wire (the operating wire 160B) becomes the predetermined value or more, the area fixed to the tense operating wire (the operating wire 160B) in the distal end portion 111 of the catheter shaft 110 is pulled to the proximal end side, and the distal end portion 111 of the catheter shaft 110 is bent as illustrated in Fig. 5. Thus, according to the present embodiment, the operating wire 160 closest to the portion shifted from the protruding portion 143 of the second rotary member 140 by 180° in the circumferential direction of the catheter shaft 110 is selected as the tense operating wire.

Further in that state, when the first rotary member 150 is rotated and the first rotary member 150 is further moved to the distal end side, the second rotary member 140 is further moved to the distal end side, there is an increase in the degree of inclination (the amount of inclination) of the swing member 130 with respect to the axis Ax, and as a result there is an increase in the degree of bending (the amount of bending) of the distal end portion 111 of the catheter shaft 110. Conversely, when the first rotary member 150 is rotated in the opposite direction and thus the first rotary member 150 is moved to the proximal end side, the second rotary member 140 is moved to the proximal end side, there is a decrease in the degree of inclination of the swing member 130, and as a result there is a decrease in the degree of bending of the distal end portion 111 of the catheter shaft 110. Thus, in the catheter 100 according to the present embodiment, the first rotary member 150 makes relative rotation with respect to the catheter shaft 110 so that the degree of bending of the distal end portion 111 of the catheter shaft 110 may be changed in a flexible manner.

As illustrated in Fig. 6, for example, when the second rotary member 140 makes relative rotation by 180° with respect to the catheter shaft 110 in the state illustrated in Fig. 5, the contact portion and the opposite portion in the swing member 130 are reversed. Accordingly, the relationship between the tense operating wire and the loosened operating wires is also reversed. That is, in the example of Fig. 6, the operating wire 160A is the tense operating wire and the operating wire 160B is the loosened operating wire. Therefore, the tensile force of the tense operating wire (the operating wire 160A) is the predetermined value or more, the area fixed to the tense operating wire (the operating wire 160A) in the distal end portion 111 of the catheter shaft 110 is pulled to the proximal end side, and the distal end portion 111 of the catheter shaft 110 is bent in the direction opposite to the direction illustrated in Fig. 5. Thus, in the catheter 100 according to the present embodiment, the second rotary member 140 makes relative rotation with respect to the catheter shaft 110 so that the bending direction of the distal end portion 111 of the catheter shaft 110 may be changed in a flexible manner. Specifically, when the second rotary member 140 makes relative rotation with respect to the catheter shaft 110, the positions of the contact portion (the portion in contact with the protruding portion 143 of the second rotary member 140) and the opposite portion (the portion shifted with respect to the contact portion by 180° in the circumferential direction of the catheter shaft 110) in the swing member 130 are changed, the operating wire 160 fixed closest to the opposite portion in the swing member 130 is selected as the tense operating wire, the area fixed to the tense operating wire in the distal end portion 111 of the catheter shaft 110 is pulled to the proximal end side, and thus the bending direction of the distal end portion 111 of the catheter shaft 110 is changed.

Even in the state where the circumferential position of the protruding portion 143 of the second rotary member 140 does not match the circumferential position of any of the four operating wires 160 at the fixed position when viewed in the direction of the axis Ax, the bending of the distal end portion 111 of the catheter shaft 110 may be achieved such that, among the four operating wires 160, the one or more operating wires 160 fixed closest to the contact portion in the swing member 130 are loosened operating wires and the one or more operating wires 160 fixed closest to the opposite portion in the swing member 130 are tense operating wire. That is, the bending direction of the distal end portion 111 of the catheter shaft 110 is not limited to the four directions corresponding to the fixed positions of the four operating wires 160 but may be set in any direction.

### A-3. Effect of First Embodiment:

As described above, the catheter 100 according to the first embodiment includes the catheter shaft 110, the operating portion 102 coupled to the proximal end portion 112 of the catheter shaft 110, and at least the one operating wire 160. The distal end portion of each of the operating wires 160 is fixed to the distal end portion 111 of the catheter shaft 110, and the proximal end portion of each of the operating wires 160 is fixed to the operating portion 102. The operating portion 102 includes the first rotary member 150. The first rotary member 150 makes relative rotation with respect to the catheter shaft 110 around the axis Ax parallel to the extending direction of the proximal end portion 112 of the catheter shaft 110 to change the tensile force of the operating wire 160 and thus change the degree of bending of the distal end portion 111 of the catheter shaft 110.

Thus, in the catheter 100 according to the first embodiment, the first rotary member 150 makes relative rotation with respect to the catheter shaft 110 so that the degree of bending of the distal end portion 111 of the catheter shaft 110 may be changed in a flexible manner. In the catheter 100 according to the first embodiment, the operating portion 102 does not include a portion such as a knob protruding laterally from the operating portion 102, and there is no portion protruding laterally from the operating portion 102 in accordance with the operation to change the degree of bending of the distal end portion 111 of the catheter shaft 110 (the relative rotation operation of the first rotary member 150). Therefore, the catheter 100 according to the first embodiment prevents a part of the operating portion 102 from being in contact with the patient's body even when the operation is performed to change the degree of bending of the distal end portion 111 of the catheter shaft 110 and when the entire catheter 100 is rotated. Therefore, with the catheter 100 according to the first embodiment, it is possible to control the degree of bending of the distal end portion 111 of the catheter shaft 110 and prevent the accompanying reduction in the operability of the catheter 100.

The catheter 100 according to the first embodiment includes the operating wires 160. The respective distal end portions of the operating wires 160 are fixed to different positions from each other in the circumferential direction in the distal end portion 111 of the catheter shaft 110. The operating portion 102 further includes the second rotary member 140. The second rotary member 140 makes relative rotation with respect to the catheter shaft 110 around the axis Ax to select the operating wire 160 having the largest tensile force from the operating wires 160 and thus change the bending direction of the distal end portion 111 of the catheter shaft 110. Specifically, the operating wire 160 closest to the portion shifted by 180° from the protruding portion 143 of the second rotary member 140 in the circumferential direction of the catheter shaft 110 is selected as the tense operating wire, and the area fixed to the tense operating wire in the distal end portion 111 of the catheter shaft 110 is pulled to the proximal end side, and thus the bending direction of the distal end portion 111 of the catheter shaft 110 is changed. Thus, in the catheter 100 according to the first embodiment, the second rotary member 140 makes relative rotation with respect to the catheter shaft 110 so that the bending direction of the distal end portion 111 of the catheter shaft 110 may be changed in a flexible manner. In the catheter 100 according to the first embodiment, there is no portion protruding laterally from the operating portion 102 in accordance with the operation to change the bending direction of the distal end portion 111 of the catheter shaft 110 (the relative rotation operation of the second rotary member 140). Therefore, the catheter 100 according to the first embodiment prevents a part of the operating portion 102 from being in contact with the patient's body even when the operation is performed to change the bending direction of the distal end portion 111 of the catheter shaft 110. Therefore, with the catheter 100 according to the first embodiment, it is possible to control the bending direction of the distal end portion 111 of the catheter shaft 110 and prevent the accompanying reduction in the operability of the catheter 100. Moreover, with the catheter 100 according to the first embodiment, the degree of bending of the distal end portion 111 of the catheter shaft 110 may be controlled by the relative rotation of the first rotary member 150, and the bending direction of the distal end portion 111 of the catheter shaft 110 may be controlled by the relative rotation of the second rotary member 140, and therefore the bending degree and the bending direction of the distal end portion 111 of the catheter shaft 110 may be controlled separately by the two rotary members, which may reduce the allowance for each control and further improve the operability of the catheter 100 as compared to a configuration in which the bending degree and the bending direction are controlled by one common operating member.

In the catheter 100 according to the first embodiment, the operating wires 160 include at least one pair of the two operating wires 160 having the distal end portions fixed to different positions from each other by 180° in the circumferential direction in the distal end portion 111 of the catheter shaft 110. Therefore, with the catheter 100 according to the first embodiment, the bending direction of the distal end portion 111 of the catheter shaft 110 may be changed to one direction and the direction opposite to the one direction by 180°, which may further improve the operability of the catheter 100.

In the catheter 100 according to the first embodiment, the operating portion 102 further includes the swing member 130. The swing member 130 is attached to the proximal end portion 112 of the catheter shaft 110 so as to change the inclination with respect to the axis Ax. The proximal end portion of the operating wire 160 is fixed to the swing member 130. The first rotary member 150 makes relative rotation to change the degree of inclination of the swing member 130 and change the tensile force of the operating wire 160. Therefore, in the catheter 100 according to the first embodiment, a relatively simple configuration enables high-accurate control on the degree of bending of the distal end portion 111 of the catheter shaft 110 by the relative rotation of the first rotary member 150. Moreover, in the catheter 100 according to the first embodiment, a change in the degree of inclination of the swing member 130 simultaneously causes an increase (tenseness) in the tensile force of one of the operating wires 160 and a decrease (looseness) in the tensile force of the other operating wire 160, and therefore the bending of the distal end portion 111 of the catheter shaft 110 may be achieved with a smaller amount of operation (a smaller amount of rotation of the first rotary member 150), and the operability of the catheter 100 may be further improved.

In the catheter 100 according to the first embodiment, the second rotary member 140 is located further on the proximal end side than the swing member 130 and is attached so as to make relative movement with respect to the catheter shaft 110 along the axis Ax. The surface of the second rotary member 140 on the side facing the swing member 130 is provided with the protruding portion 143 at a position shifted from the axis Ax. The first rotary member 150 makes relative rotation to change the position of the second rotary member 140 along the direction of the axis Ax and thus change the degree of inclination of the swing member 130. Therefore, with the catheter 100 according to the first embodiment, a relatively simple configuration enables high-accurate control on the bending degree and the bending direction of the distal end portion 111 of the catheter shaft 110 by the relative rotation of the first rotary member 150 and the second rotary member 140.

In the catheter 100 according to the first embodiment, as described above, the first rotary member 150 makes relative rotation with respect to the catheter shaft 110 so that the degree of bending of the distal end portion 111 of the catheter shaft 110 may be changed, and therefore, without further operation performed on the first rotary member 150, the bent state of the distal end portion 111 of the catheter shaft 110 may be kept, and as a result, the operator may lose hold on the operating portion 102 to, for example, handle a different combined device, changing the degree of bending of the distal end portion 111 of the catheter shaft 110 and keeping the bent state may be achieved by the identical operation, and the operability of the catheter 100 may be further improved. Moreover, in the catheter 100 according to the first embodiment, as described above, the second rotary member 140 makes relative rotation with respect to the catheter shaft 110 so that the bending direction of the distal end portion 111 of the catheter shaft 110 may be changed, and therefore by performing the operation on the second rotary member 140 without performing any operation on the first rotary member 150, the bending direction of the distal end portion 111 may be changed while the bent state of the distal end portion 111 of the catheter shaft 110 at the desired bending degree is kept, and the operability of the catheter 100 may be further improved.

### B. Second Embodiment:

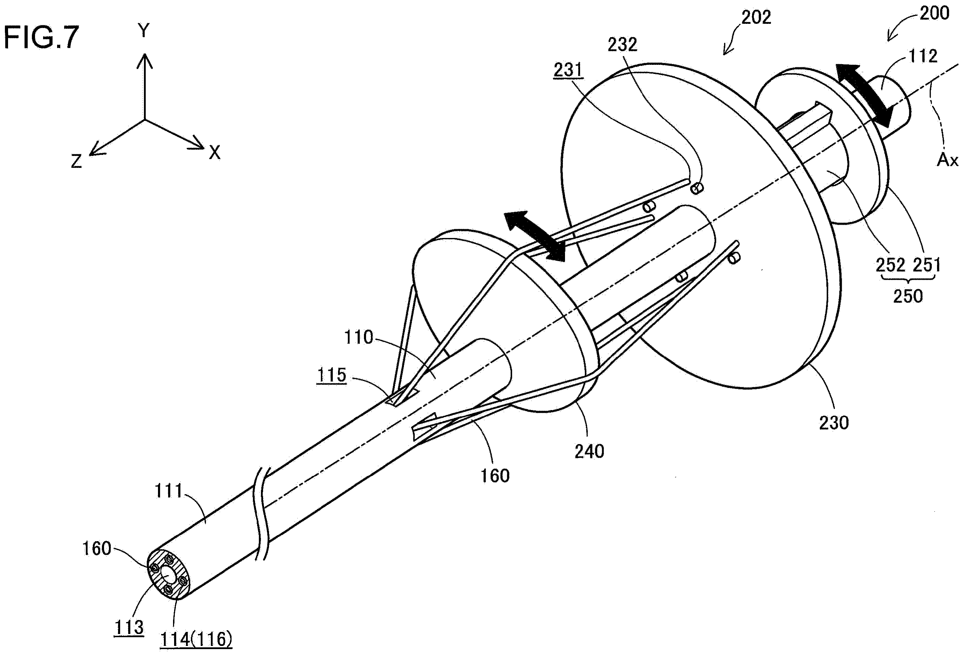

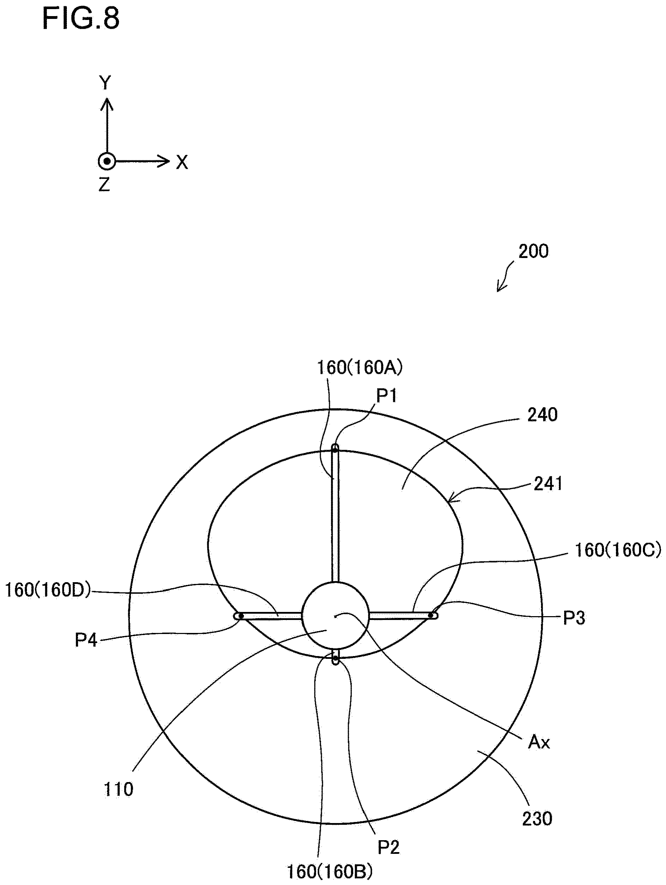

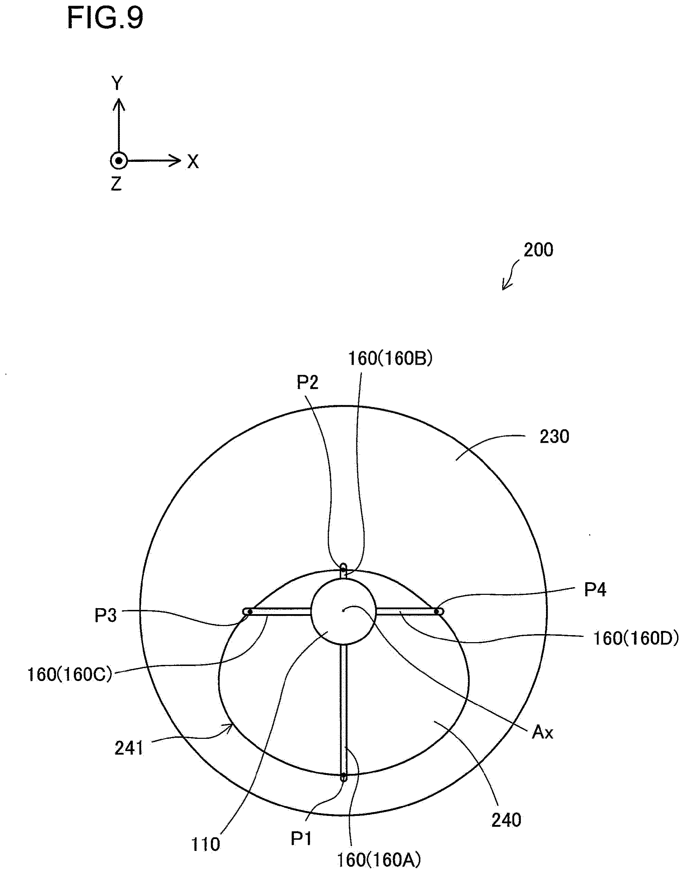

Figs. 7 to 9 are explanatory diagrams schematically illustrating a configuration of a catheter 200 according to a second embodiment. Fig. 7 illustrates an external configuration of the entire catheter 200. Figs. 8 and 9 illustrate an external configuration (a part of the configuration not illustrated) of the catheter 200 when viewed from the distal end side of the catheter 200. Hereafter, in the configuration of the catheter 200 according to the second embodiment, the same configuration as that of the catheter 100 according to the first embodiment described above is denoted by the same reference numeral, and the description thereof is omitted as appropriate.

The catheter 200 according to the second embodiment includes the catheter shaft 110, an operating portion 202, and the four operating wires 160. In Fig. 7, a part of the catheter shaft 110 is not illustrated. The configurations of the catheter shaft 110 and the operating wire 160 are the same as those in the first embodiment described above.

The operating portion 202 is a portion to perform the operation to bend the distal end portion 111 of the catheter shaft 110 and is coupled to the proximal end portion 112 of the catheter shaft 110. The operating portion 202 includes a first rotary member 250, a second rotary member 240, and a fixing member 230. The material for forming each portion included in the operating portion 202 is the same as the material for forming each portion included in the operating portion 102 according to the first embodiment described above.

The fixing member 230 is a member having substantially a disc-like shape and is attached to the proximal end portion 112 of the catheter shaft 110 so as to make relative movement with respect to the catheter shaft 110 along the axis Ax. The proximal end portion of each of the operating wires 160 is fixed to the fixing member 230. More specifically, the fixing member 230 is provided with four wire insertion holes 231 that are through-holes through which the operating wires 160 are inserted. The four wire insertion holes 231 are formed at positions shifted by 90° along the circumferential direction of the catheter shaft 110 to correspond to the four wire extraction holes 115 formed on the side surface of the catheter shaft 110. The proximal end portion of each of the operating wires 160 is inserted into the corresponding wire insertion hole 231 among the four wire insertion holes 231 formed in the fixing member 230, then folded back, inserted into another wire insertion hole, not illustrated, formed in the fixing member 230, and fixed to the fixing member 230 by a fixing tool 232 in the state where the initial tensile force has been adjusted. The surface of the fixing member 230 on the distal end side is substantially perpendicular to the axis Ax.

The second rotary member 240 is a plate-like member and is located further on the distal end side than the fixing member 230 in the proximal end portion 112 of the catheter shaft 110. The second rotary member 240 is attached so as to make relative rotation with respect to the catheter shaft 110 around the axis Ax.

As illustrated in Figs. 8 and 9, the shape of the second rotary member 240 when viewed from the distal end side of the catheter shaft 110 is the shape having an outer peripheral line 241 including at least two points having different distances from the axis Ax from each other. For example, in Figs. 8 and 9, the distance between the axis Ax and each point on the outer peripheral line 241 of the second rotary member 240 is the largest at P1 (hereinafter referred to as the "farthest point P1"), is the smallest at P2 (hereinafter referred to as the "nearest point P2"), and is an intermediate distance between them at P3, P4 (hereinafter referred to as the "intermediate point P3" and the "intermediate point P4"). According to the present embodiment, the shape of the outer peripheral line 241 of the second rotary member 240 when viewed from the distal end side of the catheter shaft 110 is a symmetrical shape with respect to the line connecting the farthest point P1 and the nearest point P2 and is such a shape that the distance from the axis Ax decreases from the farthest point P1 toward the nearest point P2 along the outer peripheral line 241.

The above-described outer peripheral line 241 of the second rotary member 240 abuts each of the operating wires 160. According to the present embodiment, as the second rotary member 240 is a plate-like member, each of the operating wires 160 abuts a side surface including the outer peripheral line 241 of the second rotary member 240.

The first rotary member 250 is located further on the proximal end side than the fixing member 230 in the proximal end portion 112 of the catheter shaft 110. The first rotary member 250 includes a disc portion 251 having substantially a disc-like shape and a hollow cylindrical portion 252 that has substantially a hollow cylindrical shape and is provided on the distal end side of the disc portion 251. The first rotary member 250 is provided with a through-hole, and an inner peripheral surface of the through-hole is provided with a spirally-arranged protruding portion or recessed portion. The outer peripheral surface of the catheter shaft 110 is provided with a recessed portion or protruding portion that engage with the protruding portion or recessed portion on the inner peripheral surface of the above-described through-hole of the first rotary member 250. As the first rotary member 250 and the catheter shaft 110 have this configuration, the first rotary member 250 makes relative rotation with respect to the catheter shaft 110 and thus makes relative movement with respect to the catheter shaft 110 along the axis Ax. This configuration allows the first rotary member 250 to maintain the state of having made relative movement along the axis Ax. When the first rotary member 250 makes relative movement along the axis Ax while making relative rotation with respect to the catheter shaft 110, the fixing member 230 accordingly makes relative movement along the axis Ax without making relative rotation with respect to the catheter shaft 110. The first rotary member 250 may be coupled to the fixing member 230 so as to make relative rotation with respect to the fixing member 230.

In the catheter 200 having this configuration according to the second embodiment, the second rotary member 240 and the first rotary member 250 in the operating portion 202 are operated so that the distal end portion 111 of the catheter shaft 110 may be bent in a flexible manner.

Figs. 7 and 8 illustrate the basic state of the catheter 200. In the state illustrated in Figs. 7 and 8, one (the operating wire 160A) of the four operating wires 160 abuts the farthest point P1 on the outer peripheral line 241 of the second rotary member 240, the other one (the operating wire 160B) abuts the nearest point P2 on the outer peripheral line 241 of the second rotary member 240, the other one (the operating wire 160C) abuts the intermediate point P3 on the outer peripheral line 241 of the second rotary member 240, and the other one (the operating wire 160D) abuts the intermediate point P4 on the outer peripheral line 241 of the second rotary member 240. Therefore, among the four operating wires 160, the operating wire 160A abutting the farthest point P1 has the largest tensile force, and the operating wire 160B abutting the nearest point P2 has the smallest tensile force. In the basic state illustrated in Fig. 7, the fixing member 230 is located on the relatively distal end side, no tensile force of the predetermined value or more acts on any of the four operating wires 160, and the distal end portion 111 of the catheter shaft 110 is not bent.

In the basic state, the first rotary member 250 makes relative rotation with respect to the catheter shaft 110 and thus the first rotary member 250 makes relative movement to the proximal end side with respect to the catheter shaft 110, and accordingly the fixing member 230 also makes relative movement to the proximal end side with respect to the catheter shaft 110. Here, the proximal end portion of each of the operating wires 160 is fixed to the fixing member 230. Therefore, the relative movement of the fixing member 230 to the proximal end side increases the tensile force of the four operating wires 160. As described above, among the four operating wires 160, the operating wire 160A abutting the farthest point P1 has the largest tensile force and therefore, when the tensile force of the four operating wires 160 increases, the tensile force of the operating wire 160A first becomes the predetermined value or more, the area fixed to the operating wire 160A in the distal end portion 111 of the catheter shaft 110 is pulled to the proximal end side, and the distal end portion 111 of the catheter shaft 110 is bent.

Further in that state, when the first rotary member 250 is rotated and the first rotary member 250 is further moved to the proximal end side, the fixing member 230 is further moved to the proximal end side, there is a further increase in the tensile force of the operating wire 160, and as a result there is an increase in the degree of bending (the amount of bending) of the distal end portion 111 of the catheter shaft 110. Conversely, when the first rotary member 250 is rotated in the opposite direction and thus the first rotary member 250 is moved to the distal end side, the fixing member 230 is moved to the distal end side, and there is a decrease in the tensile force of the operating wire 160, and as a result there is a decrease in the degree of bending of the distal end portion 111 of the catheter shaft 110. Thus, in the catheter 200 according to the present embodiment, the first rotary member 250 makes relative rotation with respect to the catheter shaft 110 so that the degree of bending of the distal end portion 111 of the catheter shaft 110 may be changed in a flexible manner.

As illustrated in Fig. 9, for example, when the second rotary member 240 makes relative rotation with respect to the catheter shaft 110 by 180° in the state illustrated in Fig. 8, the operating wire 160 abutting the farthest point P1 among the four operating wires 160 is switched from the operating wire 160A to the operating wire 160B. Therefore, the tensile force of the operating wire 160B becomes the predetermined value or more, the area fixed to the operating wire 160B in the distal end portion 111 of the catheter shaft 110 is pulled to the proximal end side, and the distal end portion 111 of the catheter shaft 110 is bent in the opposite direction. Thus, in the catheter 200 according to the present embodiment, the second rotary member 240 makes relative rotation with respect to the catheter shaft 110 so that the bending direction of the distal end portion 111 of the catheter shaft 110 may be changed in a flexible manner.

Even when the circumferential position of the farthest point P1 of the outer peripheral line 241 of the second rotary member 240 does not match the contact position of any of the four operating wires 160 when viewed in the direction of the axis Ax, there is an increase in the tensile force of the one or more operating wires 160 abutting closest to the farthest point P1 among the four operating wires 160, and thus the bending of the distal end portion 111 of the catheter shaft 110 is achieved. That is, the bending direction of the distal end portion 111 of the catheter shaft 110 is not limited to the four directions corresponding to the fixed positions of the four operating wires 160 but may be set in any direction.

As described above, the catheter 200 according to the second embodiment includes the catheter shaft 110, the operating portion 202 coupled to the proximal end portion 112 of the catheter shaft 110, and at least the one operating wire 160, as in the catheter 100 according to the first embodiment. The distal end portion of each of the operating wires 160 is fixed to the distal end portion 111 of the catheter shaft 110, and the proximal end portion of each of the operating wires 160 is fixed to the operating portion 202. The operating portion 202 includes the first rotary member 250. The first rotary member 250 makes relative rotation with respect to the catheter shaft 110 around the axis Ax parallel to the extending direction of the proximal end portion 112 of the catheter shaft 110 to change the tensile force of the operating wire 160 and thus change the degree of bending of the distal end portion 111 of the catheter shaft 110. Therefore, with the catheter 200 according to the second embodiment, it is possible to control the degree of bending of the distal end portion 111 of the catheter shaft 110 and prevent the accompanying reduction in the operability of the catheter 200, as in the catheter 100 according to the first embodiment.

The catheter 200 according to the second embodiment includes the operating wires 160, as in the catheter 100 according to the first embodiment. The respective distal end portions of the operating wires 160 are fixed to different positions from each other in the circumferential direction in the distal end portion 111 of the catheter shaft 110. The operating portion 202 further includes the second rotary member 240. The second rotary member 240 makes relative rotation with respect to the catheter shaft 110 around the axis Ax to select the operating wire 160 having the largest tensile force from the operating wires 160 and thus change the bending direction of the distal end portion 111 of the catheter shaft 110. Therefore, with the catheter 200 according to the second embodiment, it is possible to control the bending direction of the distal end portion 111 of the catheter shaft 110 and prevent the accompanying reduction in the operability of the catheter 200, as in the catheter 100 according to the first embodiment. With the catheter 200 according to the second embodiment, as in the catheter 100 according to the first embodiment, the bending degree and the bending direction of the distal end portion 111 of the catheter shaft 110 may be controlled separately by the two rotary members, which may reduce the allowance for each control and further improve the operability of the catheter 200 as compared to a configuration in which the bending degree and the bending direction are controlled by one common operating member.

In the catheter 200 according to the second embodiment, as in the catheter 100 according to the first embodiment, the operating wires 160 include at least one pair of the two operating wires 160 having the distal end portions fixed to different positions from each other by 180° in the circumferential direction in the distal end portion 111 of the catheter shaft 110. Therefore, with the catheter 200 according to the second embodiment, as in the catheter 100 according to the first embodiment, the bending direction of the distal end portion 111 of the catheter shaft 110 may be changed to one direction and the direction opposite to the one direction by 180°, which may further improve the operability of the catheter 200.

In the catheter 100 according to the second embodiment, the shape of the second rotary member 240 when viewed from the distal end side of the catheter shaft 110 is the shape having the outer peripheral line 241 including at least two points having different distances from the axis Ax from each other, and each of the operating wires 160 abuts the outer peripheral line 241. The operating portion 202 further includes the fixing member 230. The fixing member 230 is located further on the proximal end side than the second rotary member 240 and is attached so as to make relative movement with respect to the catheter shaft 110 along the axis Ax. The proximal end portion of the operating wire 160 is fixed to the fixing member 230. The first rotary member 250 makes relative rotation to change the position of the fixing member 230 along the direction of the axis Ax and thus change the tensile force of the operating wire 160. Therefore, with the catheter 200 according to the second embodiment, a relatively simple configuration enables high-accurate control on the bending degree and the bending direction of the distal end portion 111 of the catheter shaft 110 by the relative rotation of the first rotary member 250 and the second rotary member 240.

### C. Third Embodiment:

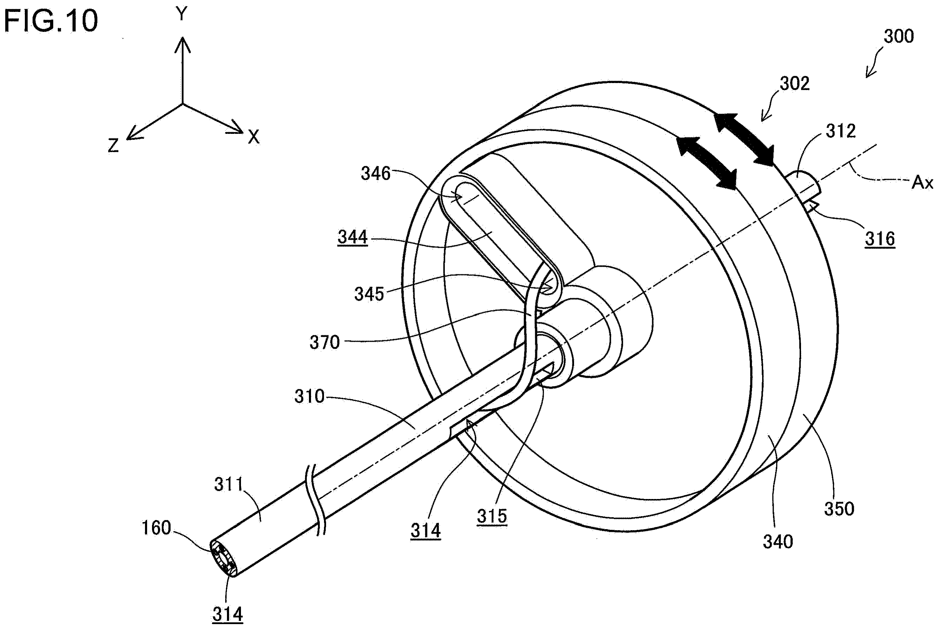

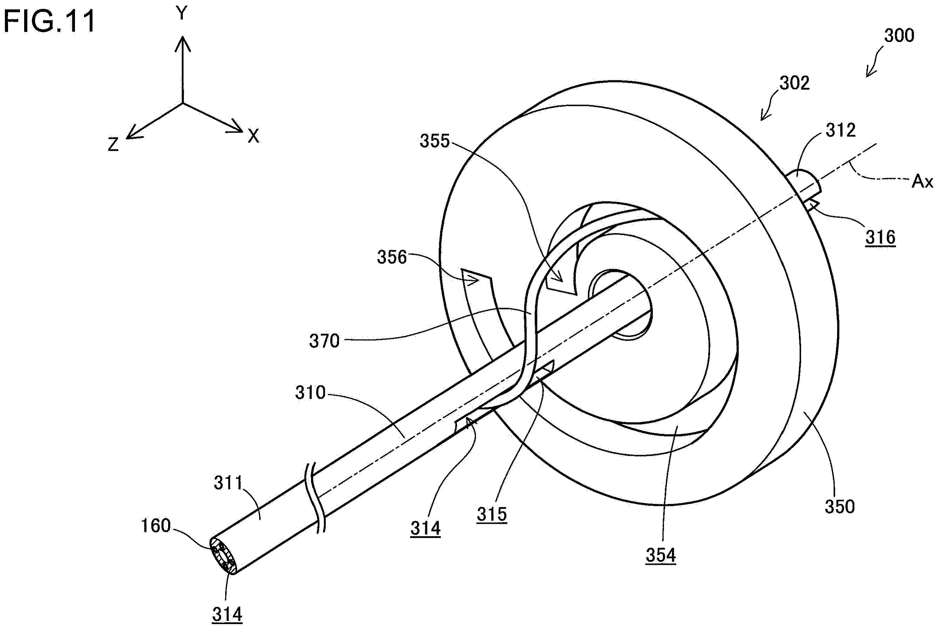

Figs. 10 and 11 are explanatory diagrams schematically illustrating a configuration of a catheter 300 according to a third embodiment. Fig. 10 illustrates an external configuration of the entire catheter 300. Fig. 11 illustrates an external configuration of the entire catheter 300, in which a second rotary member 340 described below is not illustrated. Hereafter, in the configuration of the catheter 300 according to the third embodiment, the same configuration as that of the catheter 100 according to the first embodiment described above is denoted by the same reference numeral, and the description thereof is omitted as appropriate.

The catheter 300 according to the third embodiment includes a catheter shaft 310, an operating portion 302, the four operating wires 160, and a tube member 370. In Figs. 10 and 11, part of the catheter shaft 310 is not illustrated.

The configuration of the operating wire 160 is the same as the configuration of the operating wire 160 according to the first embodiment. According to the third embodiment, however, the four operating wires 160 are accommodated in the tube member 370. The tube member 370 is a flexible tubular member. The tube member 370 is made of, for example, a resin material. Examples of the resin material for forming the tube member 370 may include polyamide resin, polyolefin resin, polyester resin, polyurethane resin, silicone resin, and fluorine resin.

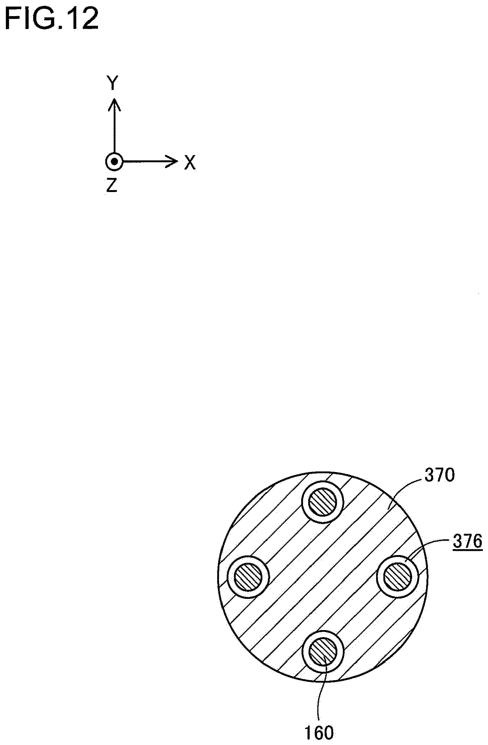

Fig. 12 is an explanatory diagram illustrating a cross-sectional configuration of the tube member 370. As illustrated in Fig. 12, the tube member 370 is provided with four through-holes 376 extending in the axial direction. In cross-section of the tube member 370, each of the through-holes 376 is circular and is formed at a position shifted by 90° along a circumferential direction of the tube member 370. Each of the through-holes 376 forms a lumen that individually accommodates the respective operating wires 160.

The configuration of the catheter shaft 310 is the same as the configuration of the catheter shaft 110 according to the first embodiment. However, according to the third embodiment, the tube member 370 is accommodated in a through-hole 314 formed in the catheter shaft 310. A side surface of a proximal end portion 312 of the catheter shaft 310 is provided with a first cable insertion hole 315 and a second cable insertion hole 316. The tube member 370 accommodated in the through-hole 314 is extracted out of the catheter shaft 310 through the first cable insertion hole 315, accommodated inside the catheter shaft 310 through the second cable insertion hole 316, and fixed to the catheter shaft 310.

The operating portion 302 is a portion to perform the operation to bend a distal end portion 311 of the catheter shaft 310 and is coupled to the proximal end portion 312 of the catheter shaft 310. The operating portion 302 includes a first rotary member 350 and the second rotary member 340. The material for forming each portion included in the operating portion 302 is the same as the material for forming each portion included in the operating portion 102 according to the first embodiment described above.

The first rotary member 350 is a member having substantially a disc-like shape and is attached so as to make relative rotation with respect to the catheter shaft 310 and the second rotary member 340 around the axis Ax parallel to the extending direction of the proximal end portion 312 of the catheter shaft 310. The first rotary member 350 is provided with a first through-hole 354. As illustrated in Fig. 11, when viewed in the direction of the axis Ax, the first through-hole 354 of the first rotary member 350 has such a shape (what is called "spiral shape") that the distance from the axis Ax gradually decreases along the circumferential direction of the catheter shaft 310 from one end (hereinafter referred to as "outer peripheral side end 356") of the first through-hole 354 to the other end (hereinafter referred to as "center side end 355"). The first through-hole 354 extends entirely (360°) in the circumferential direction of the catheter shaft 310.

The second rotary member 340 is a member having substantially a disc-like shape and is attached so as to make relative rotation with respect to the catheter shaft 310 and the first rotary member 350 around the axis Ax. The second rotary member 340 is located further on the distal end side than the first rotary member 350. The second rotary member 340 is provided with a second through-hole 344. As illustrated in Fig. 10, when viewed in the direction of the axis Ax, the second through-hole 344 of the second rotary member 340 has a shape (linear shape) extending in the radial direction of the catheter shaft 310 from one end (hereinafter referred to as "outer peripheral side end 346") of the second through-hole 344 to the other end (hereinafter referred to as "center side end 345"). According to the present embodiment, the second rotary member 340 is located further on the distal end side than the first rotary member 350, but the second rotary member 340 may be located further on the proximal end side than the first rotary member 350.

The portion of the tube member 370 extracted to the outside through the first cable insertion hole 315 of the catheter shaft 310 is inserted into the second through-hole 344 formed in the second rotary member 340 and the first through-hole 354 formed in the first rotary member 350.

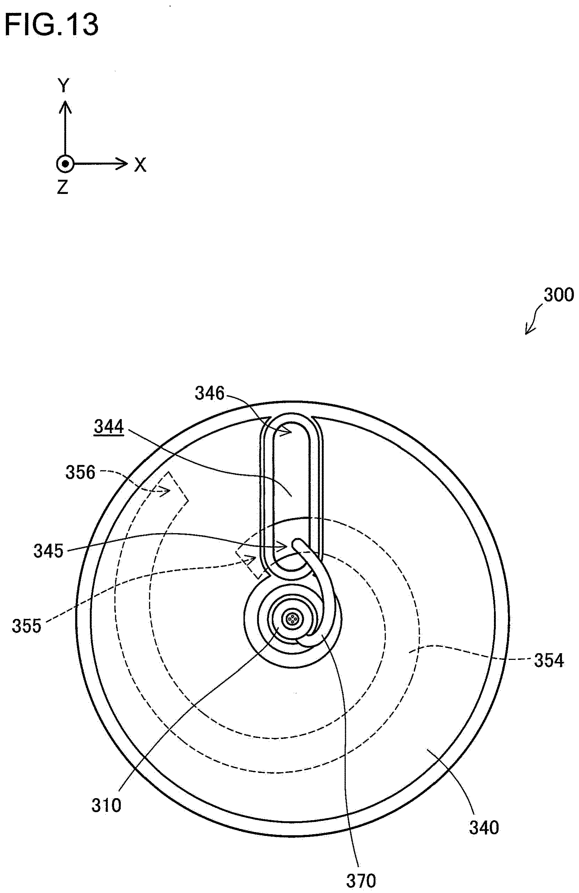

With the catheter 300 having this configuration according to the third embodiment, the second rotary member 340 and the first rotary member 350 of the operating portion 302 are operated so that the distal end portion 311 of the catheter shaft 310 may be bent in a flexible manner. Figs. 13 to 15 are diagrams illustrating an external configuration (a part of the configuration not illustrated) of the catheter 300 when viewed from the distal end side of the catheter 300 and are explanatory diagrams illustrating an operation of the catheter 300 according to the third embodiment.

Fig. 13 illustrates a basic state of the catheter 300. In this state, the relationship between the first rotary member 350 and the second rotary member 340 is such a relationship that the vicinity of the center side end 355 of the first through-hole 354 formed in the first rotary member 350 is overlapped with the second through-hole 344 formed in the second rotary member 340 when viewed in the direction of the axis Ax. Therefore, the portion of the tube member 370 inserted into the first through-hole 354 and the second through-hole 344 is located at a position relatively close to the catheter shaft 310. In this state, the tensile force of the predetermined value or more does not act on any of the four operating wires 160, and the distal end portion 311 of the catheter shaft 310 is not bent.

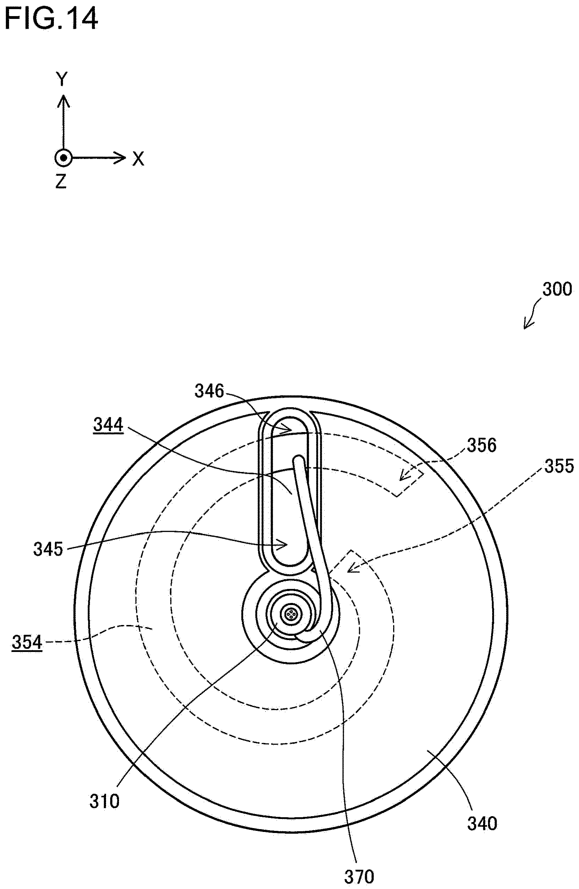

For example, as illustrated in Fig. 14, when the first rotary member 350 makes relative rotation with respect to the catheter shaft 310 and the second rotary member 340 in the basic state illustrated in Fig. 13, the positional relationship between the first through-hole 354 formed in the first rotary member 350 and the second through-hole 344 formed in the second rotary member 340 changes. In the example of Fig. 14, when viewed in the direction of the axis Ax, the relationship is such that the portion near the outer peripheral side end 356 of the first through-hole 354 is overlapped with the second through-hole 344. In accordance with this change in the positional relationship, the portion of the tube member 370 inserted into the first through-hole 354 and the second through-hole 344 moves to a position relatively far from the catheter shaft 310 in the radial direction. Thus, the tensile force of each of the operating wires 160 accommodated in each of the through-holes 376 (see Fig. 12) of the tube member 370 changes due to the difference in the distance from the axis Ax. Specifically, there is an increase in the tensile force of the operating wire 160 accommodated in the through-hole 376 formed at a position closest to the outer peripheral side in the portion of the tube member 370 inserted into the first through-hole 354 and the second through-hole 344, and the tensile force of the operating wire 160 becomes the predetermined value or more. As a result, the area fixed to the operating wire 160 in the distal end portion 311 of the catheter shaft 310 is pulled to the proximal end side, and the distal end portion 311 of the catheter shaft 310 is bent.

When the first rotary member 350 is further rotated in the state illustrated in Fig. 14 to obtain such a relationship that the outer peripheral side end 356 of the first through-hole 354 is overlapped with the second through-hole 344 when viewed in the direction of the axis Ax, the tensile force of the above-described operating wire 160 further increases and as a result the degree of bending (amount of bending) of the distal end portion 311 of the catheter shaft 310 increases. Conversely, when the first rotary member 350 is rotated in the opposite direction to obtain such a relationship that the vicinity of an intermediate position between the outer peripheral side end 356 and the center side end 355 in the first through-hole 354 is overlapped with the second through-hole 344 when viewed in the direction of the axis Ax, the tensile force of the above-described operating wire 160 decreases and as a result the degree of bending of the distal end portion 311 of the catheter shaft 310 decreases. Thus, in the catheter 300 according to the present embodiment, the first rotary member 350 makes relative rotation with respect to the catheter shaft 310 so that the degree of bending of the distal end portion 311 of the catheter shaft 310 may be changed in a flexible manner.

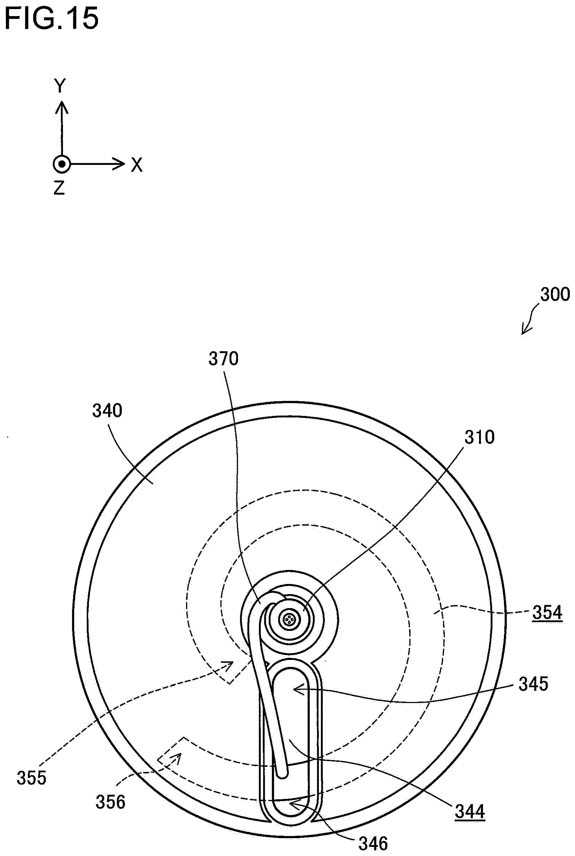

As illustrated in Fig. 15, for example, when both the first rotary member 350 and the second rotary member 340 make relative rotation with respect to the catheter shaft 310 by 180° in the state illustrated in Fig. 14, the positional relationship between the two through-holes 354, 344 and the tube member 370 is changed while the positional relationship between the first through-hole 354 formed in the first rotary member 350 and the second through-hole 344 formed in the second rotary member 340 is fixed. Accordingly, this switches the operating wire 160 having the largest tensile force among the four operating wires 160 accommodated in the tube member 370. Therefore, the tensile force of the operating wire 160 after switching becomes the predetermined value or more, the area fixed to the operating wire 160 in the distal end portion 311 of the catheter shaft 310 is pulled to the proximal end side, and the distal end portion 311 of the catheter shaft 310 is bent in the opposite direction. Thus, in the catheter 300 according to the present embodiment, both the first rotary member 350 and the second rotary member 340 make relative rotation with respect to the catheter shaft 310 so that the bending direction of the distal end portion 311 of the catheter shaft 310 may be changed in a flexible manner. The second rotary member 340 may make relative rotation with respect to the catheter shaft 310 and the first rotary member 350 so that the bending direction and the bending degree of the distal end portion 311 of the catheter shaft 310 may be changed in a flexible manner.

As described above, the catheter 300 according to the third embodiment includes the catheter shaft 310, the operating portion 302 coupled to the proximal end portion 312 of the catheter shaft 310, and at least the one operating wire 160, as in the catheter 100 according to the first embodiment. The distal end portion of each of the operating wires 160 is fixed to the distal end portion of the catheter shaft 310, and the proximal end portion of each of the operating wires 160 is fixed to the proximal end portion 312 of the catheter shaft 310. The operating portion 302 includes the first rotary member 350. The first rotary member 350 makes relative rotation with respect to the catheter shaft 310 around the axis Ax parallel to the extending direction of the proximal end portion 312 of the catheter shaft 310 to change the tensile force of the operating wire 160 and thus change the degree of bending of the distal end portion 311 of the catheter shaft 310. Therefore, with the catheter 300 according to the third embodiment, it is possible to control the degree of bending of the distal end portion 311 of the catheter shaft 310 and prevent the accompanying reduction in the operability of the catheter 300, as in the catheter 100 according to the first embodiment.

The catheter 300 according to the third embodiment includes the operating wires 160, as in the catheter 100 according to the first embodiment. The respective distal end portions of the operating wires 160 are fixed to different positions from each other in the circumferential direction in the distal end portion 311 of the catheter shaft 310. The operating portion 302 further includes the second rotary member 340. The second rotary member 340 makes relative rotation with respect to the catheter shaft 310 around the axis Ax to select the operating wire 160 having the largest tensile force from the operating wires 160 and thus change the bending direction of the distal end portion 311 of the catheter shaft 310. Therefore, with the catheter 300 according to the third embodiment, it is possible to control the bending direction of the distal end portion 311 of the catheter shaft 310 and prevent the accompanying reduction in the operability of the catheter 300, as in the catheter 100 according to the first embodiment. With the catheter 300 according to the third embodiment, as in the catheter 100 according to the first embodiment, the bending degree and the bending direction of the distal end portion 311 of the catheter shaft 310 may be controlled separately by the two rotary members, which may reduce the allowance for each control and further improve the operability of the catheter 300 as compared to a configuration in which the bending degree and the bending direction are controlled by one common operating member.

In the catheter 300 according to the third embodiment, as in the catheter 100 according to the first embodiment, the operating wires 160 include at least one pair of the two operating wires 160 having the distal end portions fixed to different positions from each other by 180° in the circumferential direction in the distal end portion 311 of the catheter shaft 310. Therefore, with the catheter 300 according to the third embodiment, as in the catheter 100 according to the first embodiment, the bending direction of the distal end portion 311 of the catheter shaft 310 may be changed to one direction and the direction opposite to the one direction by 180°, which may further improve the operability of the catheter 300.

The catheter 300 according to the third embodiment further includes the tube member 370. The tube member 370 is provided with the through-holes 376 individually accommodating the respective operating wires 160. The first rotary member 350 is provided with the first through-hole 354 penetrating in the direction of the axis Ax. The first through-hole 354 has such a shape that the distance from the axis Ax gradually decreases along the circumferential direction of the catheter shaft 310 when viewed in the direction of the axis Ax. The second rotary member 340 is provided with the second through-hole 344 penetrating in the direction of the axis Ax. The second through-hole 344 has a shape extending in the radial direction of the catheter shaft 310 when viewed in the direction of the axis Ax. The tube member 370 is inserted into the first through-hole 354 and the second through-hole 344. Therefore, with the catheter 300 according to the third embodiment, a relatively simple configuration enables high-accurate control on the bending degree and the bending direction of the distal end portion 311 of the catheter shaft 310 by the relative rotation of the first rotary member 350 and the second rotary member 340.

### D. Modification:

The technique disclosed in this description is not limited to the embodiments described above and may be modified to various embodiments without departing from the spirit thereof and for example may be modified as described below.

The configuration of the catheter according to each of the above embodiments is merely an example and may be modified in various ways. For example, the catheter according to each of the above embodiments includes two pairs of two operating wires fixed to different positions from each other by 180° in the circumferential direction in the distal end portion of the catheter shaft, but the catheter may include the only one pair, three or more pairs, or may not include the pair. The catheter according to each of the above embodiments includes the four operating wires 160, but the number of the operating wires 160 included in the catheter may be changed as appropriate. For example, the catheter according to each of the above embodiments may include the only one operating wire 160. The fixing position of each of the operating wires 160 according to each of the embodiments to the distal end portion of the catheter shaft is merely an example and may be changed as appropriate.

In the catheter according to the above embodiment, it is possible to control the bending degree and the bending direction of the distal end portion of the catheter shaft, but a configuration may be such that it is possible to control the degree of bending of the distal end portion of the catheter shaft but not possible to control the bending direction.

The material of each member according to each of the above embodiments is merely an example and may be modified in various ways.

According to the first embodiment described above, the spring member 137 and the fixing member 138 are used to enable changes in the inclination of the swing member 130, but another configuration may be used to enable changes in the inclination of the swing member 130. According to the first embodiment above, the ball 142 forms the protruding portion 143 of the second rotary member 140, but another member may form the protruding portion 143. For example, the second rotary member 140 that is an integral member having a shape including the protruding portion 143 may be used. The alignment order of the members constituting the operating portion 102 in the direction of the axis Ax according to the above first embodiment is not limited to the alignment order described above.

The shape of the second rotary member 240 according to the above second embodiment is merely an example, and any shape may be adopted as long as it is a shape including the outer peripheral line 241 including at least two points having different distances from the axis Ax from each other.

The shape of the first through-hole 354 of the first rotary member 350 according to the above third embodiment is merely an example, and any shape may be adopted as long as it is such a shape that the distance from the axis Ax gradually decreases along the circumferential direction of the catheter shaft.

### DESCRIPTION OF REFERENCE NUMERALS

100 Catheter
102 Operating portion
110 Catheter shaft
111 Distal end portion
112 Proximal end portion
113 Through-hole
114 Through-hole
115 Wire extraction hole
116 Through-hole
130 Swing member
131 Wire insertion hole
132 Fixing tool
134 Shaft insertion hole
137 Spring member
138 Fixing member
140 Second rotary member
141 Receiving portion
142 Ball
143 Protruding portion
144 Cover member
150 First rotary member
151 Disc portion
152 Hollow cylindrical portion
160 Operating wire
200 Catheter
202 Operating portion
230 Fixing member
231 Wire insertion hole
232 Fixing tool
240 Second rotary member
241 Outer peripheral line
250 First rotary member
251 Disc portion
252 Hollow cylindrical portion
300 Catheter
302 Operating portion
310 Catheter shaft
311 Distal end portion
312 Proximal end portion
314 Through-hole
315 First cable insertion hole
316 Second cable insertion hole
340 Second rotary member
344 Second through-hole
345 Center side end
346 Outer peripheral side end
350 First rotary member
354 First through-hole
355 Center side end
356 Outer peripheral side end
370 Tube member
376 Through-hole

## Claims

1. A catheter comprising:
a catheter shaft;
an operating portion coupled to a proximal end portion of the catheter shaft; and
at least one operating wire, a distal end portion of the operating wire being fixed to a distal end portion of the catheter shaft, and a proximal end portion of the operating wire being fixed to the operating portion or the proximal end portion of the catheter shaft, wherein
the operating portion includes a first rotary member that makes relative rotation with respect to the catheter shaft around an axis parallel to an extending direction of the proximal end portion of the catheter shaft to change a tensile force of the operating wire and thus change a degree of bending of the distal end portion of the catheter shaft.

2. The catheter according to claim 1, comprising a plurality of the operating wires, wherein
the respective distal end portions of the operating wires are fixed to different positions from each other in a circumferential direction in the distal end portion of the catheter shaft, and
the operating portion further includes a second rotary member that makes relative rotation with respect to the catheter shaft around the axis to select the operating wire having a largest tensile force among the operating wires and thus change a bending direction of the distal end portion of the catheter shaft.

3. The catheter according to claim 2, wherein the operating wires include at least one pair of the two operating wires having the distal end portions fixed to different positions from each other by 180° in the circumferential direction in the distal end portion of the catheter shaft.

4. The catheter according to claim 2 or 3, wherein
the operating portion further includes a swing member that is attached to the proximal end portion of the catheter shaft so as to change an inclination with respect to the axis and that has the proximal end portion of the operating wire fixed thereto, and
the first rotary member makes the relative rotation to change a degree of inclination of the swing member and change the tensile force of the operating wire.

5. The catheter according to claim 4, wherein
the second rotary member is located further on a proximal end side than the swing member and is attached so as to make relative movement with respect to the catheter shaft along the axis,
a surface of the second rotary member on a side facing the swing member is provided with a protruding portion at a position shifted from the axis, and
the first rotary member makes the relative rotation to change a position of the second rotary member along a direction of the axis and thus change the degree of inclination of the swing member.

6. The catheter according to claim 2 or 3, wherein
a shape of the second rotary member when viewed from a distal end side of the catheter shaft is a shape having an outer peripheral line including at least two points having different distances from the axis from each other, and each of the operating wires abuts the outer peripheral line,
the operating portion further includes a fixing member that is located further on a proximal end side than the second rotary member, is attached so as to make relative movement with respect to the catheter shaft along the axis, and has the proximal end portion of the operating wire fixed thereto, and
the first rotary member makes the relative rotation to change a position of the fixing member along a direction of the axis and thus change the tensile force of the operating wire.

7. The catheter according to claim 2 or 3, further comprising a tube member provided with a plurality of through-holes individually accommodating the respective operating wires, wherein
the first rotary member is provided with a first through-hole that penetrates in a direction of the axis and has such a shape that a distance from the axis gradually decreases along the circumferential direction of the catheter shaft when viewed in the direction of the axis,
the second rotary member is provided with a second through-hole that penetrates in the direction of the axis and has a shape extending in a radial direction of the catheter shaft when viewed in the direction of the axis, and
the tube member is inserted into the first through-hole and the second through-hole.
